# FASCICULE DE BREVET EUROPEEN

(11) **EP 0 625 514 B1**
(45) Date de publication et mention de la délivrance du brevet: **25.08.1999**
(21) Numéro de dépôt: 94401032.1
(22) Date de dépôt: 10.05.1994
(51) Int. Cl.: C07D 241/26, C07D 401/12, A61K 31/495

(54) **Dérivés de 2-aminopyrazine-5-carboxamide, leur préparation et leur application en thérapeutique**
2-Aminopyrazin-5-carbonsäureamid Derivate, deren Herstellung und deren Verwendung als Heilmittel
2-Amino-pyrazin-5-carboxamid derivatives, their preparation and their use in therapy

(30) Priorité: 17.05.1993 FR 9305930
(43) Date de publication de la demande: 23.11.1994
(73) Titulaire: SYNTHELABO, 92350 Le Plessis Robinson (FR)
(72) Inventeur: George, Pascal, F-78730 St Arnoult en Yvelines (FR); Marabout, Benoit, F-91300 Massy (FR); Froissant, Jacques, F-41160 Moree (FR)
(74) Mandataire: Ludwig, Jacques

(56) Documents cités:
- EP-A- 0 435 749

## Description

La présente invention a pour objet des dérivés de 2-aminopyrazine-5-carboxamide, leur préparation et leur application en thérapeutique.

Les composés de l'invention répondent à la formule générale (I) 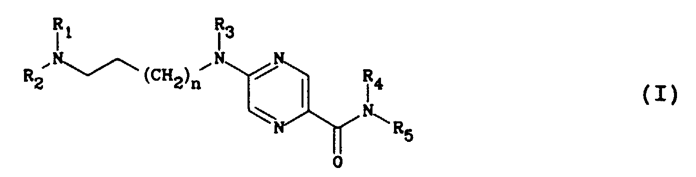 dans laquelle
n représente le nombre 0 ou 1,
R₁ représente un groupe méthyle, auquel cas
R₂ représente un groupe phénoxy(C₁-C₄)alkyle (dont le groupe phénoxy porte éventuellement 1 ou 2 substituants choisis parmi les atomes d'halogène et les groupes méthoxy et éthoxy), ou bien R₁ et R₂ forment ensemble, et avec l'atome d'azote qui les porte, un groupe 4-(phénoxyméthyl)pipéridin-1-yle (dont le groupe phénoxy porte éventuellement 1 ou 2 groupes alkyles en C₁-C₄), ou un groupe 4-phénylpipérazin-1-yle (dont le groupe phényle porte éventuellement 1 ou 2 substituants choisis parmi les atomes d'halogènes et les groupes méthoxy et éthoxy et les groupes alkyles en C₁-C₄),
R₃ représente un atome d'hydrogène ou un groupe méthyle,
R₄ représente un atome d'hydrogène,
R₅ représente un atome d'hydrogène ou un groupe de formule générale 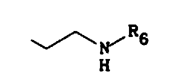
R₆ étant un atome d'hydrogène, un groupe tertiobutyloxycarbonyle, un groupe 4-carbamoylpyrimidin-2-yle ou un groupe 5-carbamoylpyrazin-2-yle.

Les composés de l'invention peuvent exister à l'état de bases ou de sels d'addition à des acides.
Conformément à l'invention, on peut préparer les composés de formule générale (I) par un procédé illustré par le schéma qui suit.

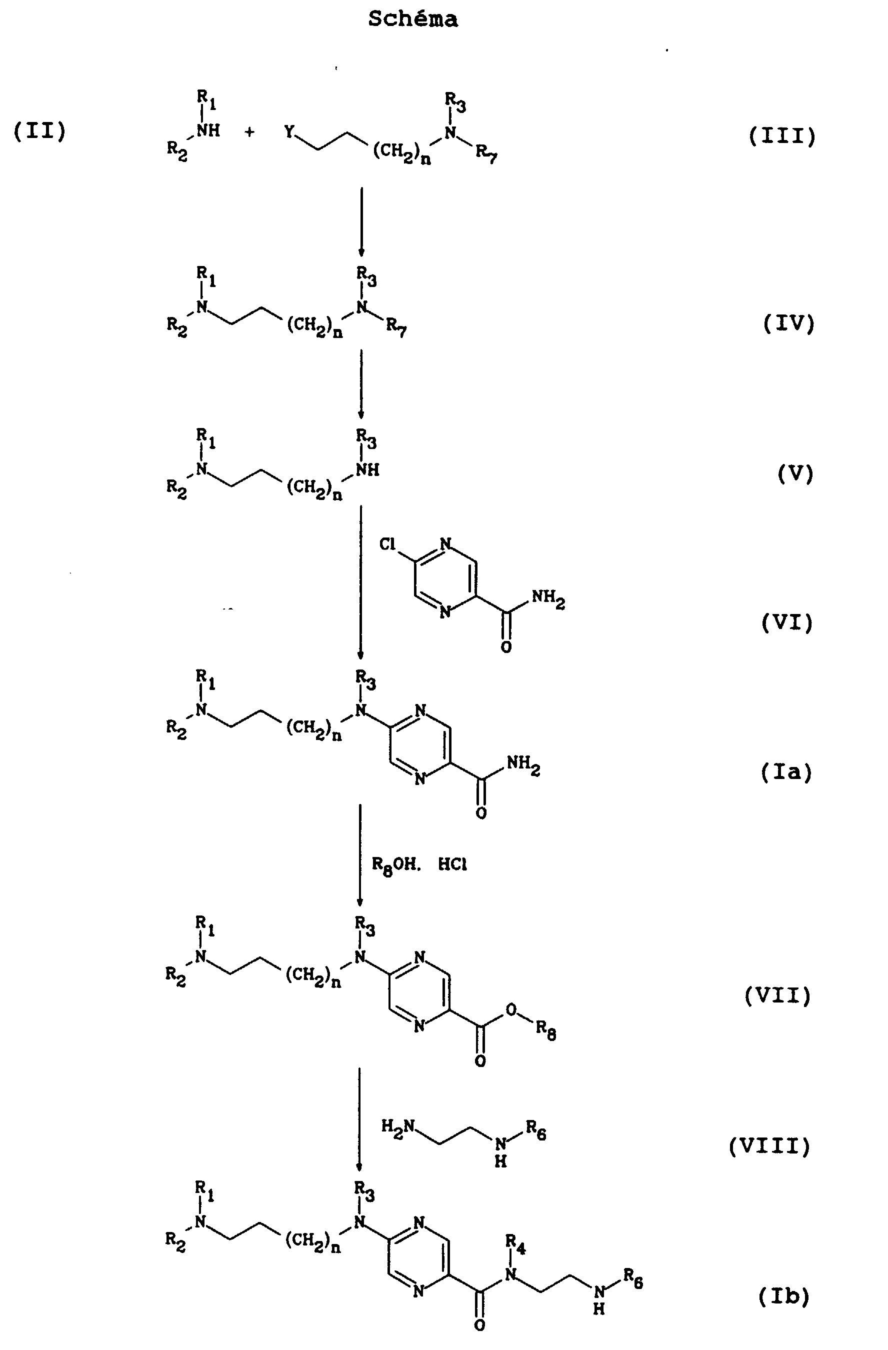

On fait réagir une amine de formule générale (II) dans laquelle R₁ et R₂ sont tels que définis ci-dessus, éventuellement sous forme de sel, avec un réactif halogéné de formule générale (III) dans laquelle Y représente un atome d'halogène, n est tel que défini ci-dessus, et soit R₃ est tel que défini ci-dessus et R₇ représente un groupe protecteur de l'amine, par exemple un groupe triphénylméthyle, soit R₃ et R₇ forment ensemble, et avec l'atome d'azote qui les porte, un groupe phtalimido, comme décrit dans *J. Med. Chem.* (1989), **32**(8), 1921-1926.
La réaction s'effectue dans un solvant aprotique tel que le diméthylformamide, en présence d'une base minérale telle que le carbonate de potassium, à une température de 40 à 80°C.

On obtient une diamine de formule générale (IV), dont on déprotège l'alkylamine terminale : dans le cas où R₇ est un groupe triphénylméthyle, on effectue un traitement à l'acide chlorydrique gazeux dans un alcool aliphatique, par exemple le méthanol, à une température de 0 à 60°C ; dans le cas où R₃ et R₇ forment ensemble un groupe phtalimido, on effectue un traitement analogue à celui décrit dans la littérature citée ci-dessus, par exemple avec l'hydrazine.

On obtient une amine de formule générale (V), que l'on fait réagir avec le 2-chloropyrazine-5-carboxamide de formule (VI) dans un solvant aprotique, par exemple le *N*,*N*-diméthylformamide, en présence d'une base, par exemple le carbonate de potassium, à une température de 20 à 40°C, pour arriver au dérivé de 2-aminopyrazine-5-carboxamide de formule générale (Ia) qui correspond à la formule générale (I) lorsque R₄ et R₅ représentent chacun un atome d'hydrogène.

Pour préparer les composés de formule générale (I) dans laquelle R₅ représente un groupe de formule générale 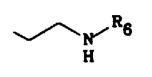 on transforme un amide de formule générale (Ia), dans laquelle n, R₁, R₂, R₃ sont tels que définis ci-dessus, en ester de formule générale (VII) dans laquelle R₈ représente un groupe alkyle en C₁-C₄, par réaction avec un alcool aliphatique en C₁-C₄, par exemple le méthanol, en présence d'un acide, par exemple l'acide chlorhydrique gazeux, à une température de 0 à 60°C, puis on fait réagir l'ester ainsi obtenu avec une diamine de formule générale (VIII), dans laquelle R₆ représente un groupe protecteur de l'amine, par exemple un groupe tertiobutyloxycarbonyle, dans un alcodl aliphatique par exemple le méthanol ou le n-butanol, à une température de 0 à 100°C, pour obtenir un composé de formule générale (Ib) dans laquelle R₆ représente un groupe tertiobutyloxycarbonyle.

Pour préparer les composés de formule générale (Ib) dans laquelle R₆ représente un groupe 4-carbamoylpyrimidin-2-yle- ou 5-carbamoylpyrazin-2-yle, on déprotège le composé précédemment obtenu selon une méthode connue, par exemple par l'acide trifluoroacétique dans le dichlorométhane, pour obtenir le composé de formule générale (Ib) où R₆ représente l'hydrogène, et l'on fait réagir ce dernier avec le 2-chloropyrimidine-4-carboxamide où le 2-chloropyrazine-5-carboxamide, dans un solvant aprotique par exemple le *N,N*-diméthylformamide, en présence d'une base, par exemple le carbonate de potassium, à une température de 20 à 40°C.

Les amines de formule générale (II) peuvent être préparées par des méthodes analogues à celles décrites dans *Bull. Soc. Chim.* (1959) 839-849 dans le cas des phénoxyalkyamines ; *J. Med. Chem.* (1987) **30**(1) 222-5 et brevet DE-2737630 dans le cas des phénoxyméthylpipéridines.

Le réactif halogéné de formule générale (III) est soit disponible dans le commerce quand R₃ et R₇ forment ensemble un groupe phtalamido, soit, quand R₃ représente H ou CH₃, peut être préparé par une méthode analogue à celle décrite dans la demande de brevet FR-2656609.

Le 2-chloropyrazine-5-carboxamide de formule (VI) peut être préparé par une méthode analogue à celle décrite dans *J. Het. Chem.* 1974, **11**, 607-610, *Agric. Biol. Chem.* 1982, **46**(8), 2169-2172, *Coll. Czech. Chem. Comm.* 1990, **50**, 2493-2501 et *Coll. Czech. Chem. Comm.* 1972, **37**, 862-867.

Le 2-chloropyrimidine-4-carboxamide peut être préparé par une méthode analogue à celle décrite dans la demande de brevet FR-2656609.

Les diamines monoprotégées de formule générale (VII) peuvent être préparées par des méthodes analogues à celles décrites dans *Synthesis* (1990) 366-368.

Les exemples suivants illustrent en détail la préparation de quelques composés selon l'invention. Les microanalyses élémentaires et les spectres IR et RMN confirment les structures des composés obtenus.
Les numéros indiqués entre parenthèses dans les titres correspondent à ceux de la première colonne du tableau donné plus loin.

### Exemple 1 (Composé n°1).

(*E*) -But-2-ènedioate de 2-[[3-[(2-(2-méthoxyphénoxy)éthyl]méthylamino]propyl]amino]pyrazine-5-carboxamide (1:1).

### 1.1. N-[2-(2-Méthoxyphénoxy)éthyl]-N-méthyl-N'-(triphénylméthyl)propane-1,3-diamine.

Dans un ballon tricol de 500 ml, on introduit sous argon, 8,05 g (0,0370 mole) de chlorhydrate de *N*-méthyl-2-(2-méthoxyphénoxy)éthylamine, 15,5 g (0,0407 mole) de *N*-triphényl-3-bromopropylamine, (0,0925 mole) de carbonate de potassium et 75 ml de *N,N*-diméthylformamide. On agite le mélange pendant 15,5h à 90°C. On traite le mélange réactionnel par un mélange d'eau et de glace et extrait à l'acétate d'éthyle. On lave la phase organique à l'eau, on la sèche sur sulfate de sodium et on la concentre sous pression réduite.
On obtient 18,2 g d'une huile orange que l'on purifie par chromatographie sur gel de silice en éluant avec un mélange 98/2 de dichlorométhane/méthanol. On obtient 13,7 g d'huile qu'on utilise telle quelle dans l'étape suivante.

### 1.2. N-[2-(2-Méthoxyphénoxy)éthyl]-N-méthylpropane-1,3-diamine.

Dans un ballon de 1 l, on introduit 12,9 g (0,0268 mole) de *N*-[2-(2-méthoxyphénoxy)éthyl]-*N*-méthyl-*N*'-(triphénylméthyl)propane-1,3-diamine et 250 ml de méthanol. On fait passer un courant d'acide chlorhydrique gazeux pendant 15 mn, en refroidissant par un mélange d'eau et de glace. On laisse revenir le mélange à la température ambiante, puis le porte à la température du reflux pendant 7,5h. On concentre le mélange à sec, reprend le résidu dans de l'éthanol et concentre à nouveau. On reprend le résidu à l'eau, on alcalinise le mélange, on reprend l'huile surnageante avec de l'acide chlorhydrique dilué et on l'extrait à l'éther diéthylique. On traite ensuite la phase aqueuse acide avec de la soude jusqu'à pH basique et on l'extrait au dichlorométhane. On lave la phase organique à l'eau, on la sèche sur sulfate de sodium et on la concentre sous pression réduite. On obtient 5,6 g d'une huile jaune qu'on utilise telle quelle dans l'étape suivante.

### 1.3. (E)-But-2-ènedioate de 2-[[3-[[2-(2-méthoxyphénoxy)éthyl]méthylamino]propyl]amino]pyrazine-5-carboxamide.

Dans un ballon de 250 ml on introduit, sous argon, 5,0 g (0,021 mole) de *N*-[2-(2-méthoxyphénoxy)éthyl]-*N*-méthylpropane-1,3-diamine, 3,3 g (0,021 mole) de 2-chloropyrazine-5-carboxamide, 100 ml d'acétonitrile et quelques cristaux d'iodure de sodium. On ajoute 2,9 g (0,021 mole) de carbonate de potassium et on chauffe le mélange à la température du reflux pendant 30h.
On refroidit à température ambiante, on collecte le précipité par filtration et on le purifie par chromatographie sur colonne de gel de silice en éluant avec un mélange dichloro-méthane/méthanol de 100/0 à 90/10.
On recristallise le solide obtenu dans l'acétonitrile et on obtient 2,82 g (0,00785 mole) de base.
On prépare le fumarate à partir de 2,82 g de base dissous dans 50 ml de méthanol, par addition de 0,91 g (0,00785 mole) d'acide fumarique en solution dans 50 ml de méthanol. On concentre la solution sous pression réduite et on recristallise dans l'éthanol. On obtient 3,32 g de solide blanc.
Point de fusion : 161-163°C.

### Exemple 2. (Composé n°3)

### Chlorhydrate de 2-[[3-[4-[[5-méthyl-2-(1-méthyléthyl)phénoxy]méthyl]pipéridin-1-yl]propyl]amino]pyrazine-5-carboxamide (1:1).

### 2.1. 2-[3-[4-[[5-Méthyl-2-(1-méthyléthyl)phénoxy]méthyl]pipéridin-1-y1]propyl]-1H-isoindole-1,3(2H)-dione.

On fait réagir 11,35 g (0,04 mole) de chlorhydrate de 4-[[5-méthyl-2-(1-méthyléthyl)phénoxy]méthyl]pipéridine, 10,72 g (0,04 mole) de 2-(3-bromopropyl)-1*H*-isoindole-1,3(2*H*)-dione et 13,8 g (0,1 mole) de carbonate de potassium dans 113 ml de *N,N*-diméthylformamide. On agite le mélange pendant 3h à 100°C. On le verse dans de l'eau glacée. On extrait la solution à l'acétate d'éthyle et on la lave à l'eau. On sèche la phase organique sur sulfate de sodium, on la filtre et on la concentre sous pression réduite. On utilise le produit obtenu tel quel dans l'étape suivante.

### 2.2. 4-[[5-Méthyl-2-(1-méthyléthyl)phénoxy]méthyl]pipéridine-1-propanamine.

On fait réagir 17,35 g (0,04 mole) de 2-[3-[4-[[5-méthyl-2-(1-méthyléthyl)phénoxy]méthyl]pipéridin-1-yl]propyl]-1*H*-isoindole-1,3(2*H*)-dione dans 340 ml d'éthanol avec 3,9 ml (0,08 mole) d'hydrate d'hydrazine. On chauffe le mélange à la température du reflux pendant 3h. On filtre, en rinçant le solide avec un peu d'éthanol, on concentre le filtrat et on le reprend dans de l'éther diéthylique. On élimine à nouveau un insoluble par filtration et on concentre à nouveau le filtrat. On réunit les insolubles dans un ballon et on ajoute 25 ml d'acide chlorhydrique concentré et 75 ml d'eau. Tout en agitant on porte au reflux pendant 2h. On laisse refroidir, on élimine l'insoluble par filtration, on rince avec de l'eau, on alcalinise avec de l'ammoniaque concentrée, et on extrait trois fois à l'éther diéthylique. On sèche la phase organique sur sulfate de sodium, on la filtre et on la concentre sous pression réduite. On obtient un composé qu'on utilise tel quel dans l'étape suivante.

### 2.3. Chlorhydrate de 2-[[3-[4-[[5-méthyl-2-(1-méthyléthyl)phénoxy]méthyl]pipéridin-1-yl]propyl]amino]pyrazine-5-carboxamide (1:1).

On fait réagir 7,45 g (0,0245 mole) de 4-[[5-méthyl-2-(1-méthyléthyl)phénoxy]méthyl]pipéridine-1-propanamine, 3,86 g (0,0245 mole) de 2-chloropyrazine-5-carboxamide et 3,38 g (0,0246 mole) de carbonate de potassium dans 100 ml d'acétonitrile. On chauffe le mélange pendant 28h à la température du reflux puis on laisse refroidir à température ambiante et on évapore le solvant sous pression réduite.
on purifie le solide obtenu par chromatographie sur colonne de gel de silice en éluant avec un mélange dichlorométhane/méthanol de 100/0 à 80/20. On recristallise le solide obtenu dans l'acétate d'éthyle et on obtient 1,07 g (0,0025 mole) de base.
On prépare le chlorhydrate à partir de 1,07 g de base en solution dans 20 ml de propan-2-ol, par addition de 25 ml d'acide chlorhydrique 0,1N dans le propan-2-ol, puis on évapore le solvant sous pression réduite. On recristallise le résidu dans le propan-2-ol pour obtenir finalement 0,7 g de solide blanc.
Point de fusion : 218-220°C.

### Exemple 3 (Composé n°5)

(*E*) -but-2-ènedioate de 2-[[2-[4-(2-méthoxyphényl)pipérazin-1-yl]éthyl]amino]pyrazine-5-carboxamide (1:1).

### 3.1. 2-[4-(2-Méthoxyphényl)pipérazin-1-yl]-N-(triphénylméthyl)éthanamine.

Dans un ballon de 500 ml muni d'un réfrigérant et placé sous azote on introduit 10 g (0,273 mole) de 2-bromo-*N*-(triphénylméthyl)éthanamine, 200 ml d'acétonitrile, 5,15 g (0,0273 mole) de 1-(2-méthoxyphényl)pipérazine, 5,6 g de carbonate de potassium anhydre , quelques grains d'iodure de sodium, et 1 ml de diméthylformamide. On chauffe le mélange au reflux pendant 15h, on évapore les solvants, on ajoute de l'eau et du dichlorométhane, on sépare la phase organique, on la lave à l'eau, on la sèche sur sulfate de sodium et on évapore le solvant sous pression réduite. On obtient une huile visqueuse qu'on purifie par chromatographie sur colonne de gel de silice en éluant avec un mélange d'acétate d'éthyle et de dichlorométhane. On isole 9,24 g de produit qu'on utilise tel quel dans l'étape suivante.

### 3.2. 2-[4-(2-Méthoxyphényl)pipérazin-1-yl]éthanamine, trichlorhydrate.

On dissout 9,24 g de 2-[4-(2-méthoxyphényl)pipérazin-1-yl]-*N*-(triphénylméthyl)éthanamine dans 400 ml de méthanol et, après homogénéisation, on fait passer dans la solution un courant d'acide chlorhydrique gazeux pendant 10mn. On collecte le précipité, on le rince au méthanol et on le sèche sous vide. On obtient 5,33 g de solide blanc.

### 3.3. (E) but-2-ènedioate de 2-[[2-[4-(2-méthoxyphényl)pipérazin-1-yl]éthyl]amino]pyrazine-5-carboxamide.

Dans un ballon de 500 ml muni d'un réfrigérant et placé sous azote on introduit 5,7 g (0,0242 mole) de 2-[4-(2-méthoxyphényl)pipérazin-1-yl]éthanamine, 3,82 g (0,0242 mole) de 2-chloropyrazine-5-carboxamide, 200 ml d'acétonitrile et 3,35 g (0,0242 mole) de carbonate de sodium. On chauffe le mélange au reflux pendant 22h, on le laisse refroidir, on évapore le solvant sous pression réduite. On purifie le résidu par chromatographie sur colonne de gel de silice en éluant avec un mélange dichlorométhane/méthanol de 100/0 à 85/15, et on recristallise le solide obtenu dans l'acétate d'éthyle. On obtient 0,96 g (0,0027 mole) de base.
On prépare le fumarate à partir de 0,96 g de base en solution dans 50 ml de méthanol et de 0,31 g (0,0027 mole) d'acide fumarique en solution dans 50 ml de méthanol. On concentre le mélange sous pression réduite et le produit cristallise. On obtient 0,97 g de solide blanc.
Point de fusion : 220-222°C.

### Exemple 4 (Composé n°12)

(*E*)-but-2-ènedioate de 2-[[3-[4-(2-cyclopropylphényl)pipérazin-1-yl]propyl]méthylamino]pyrazine-5-carboxamide (1:1).

### 4.1. 3-[4-(2-Cyclopropylphényl)pipérazin-1-yl]-N-méthylpropanamine, trichlorhydrate.

Dans un ballon de 500 ml muni d'un réfrigérant et placé sous azote on introduit 9,0 g (0,0444 mole) de 1-(2-cyclopropylphényl)pipérazine, 200 ml de diméthylformamide, 17,5 g (0,0444 mole) de 3-bromo-*N*-méthyl-*N*-(triphénylméthyl)propanamine et 9 g de carbonate de potassium, et on chauffe le mélange trois fois pendant 6h à 96°C. On évapore le solvant sous pression réduite, on reprend le résidu avec de l'eau et du dichlorométhane, on sépare la phase organique, on la lave à l'eau, on la sèche sur sulfate de sodium et on évapore le solvant sous pression réduite. On obtient 4,17 g de 3-[4-(2-cyclopropylphényl)pipérazin-1-yl]-*N*-méthyl-*N*-(triphénylméthyl)propanamine sous forme d'huile qu'on dissout dans 200 ml de méthanol, on y fait passer un courant d'acide chlorhydrique gazeux pendant 10mn, on concentre le mélange, on le laisse reposer 2 jours et on sépare le précipité par filtration. On obtient 2,94 g de composé.

### 4.2. (E) -but-2-ènedioate de 2-[[3-[4-(2-cyclopropylphényl)pipérazin-1-yl]propyl]méthylamino]pyrazine-5-carboxamide (1:1).

Dans un ballon de 500 ml muni d'un réfrigérant et placé sous azote on introduit 3,77 g (0,0138 mole) de 3-[4-(2-cyclopropylphényl)pipérazin-1-yl]-*N*-méthylpropanamine, 2,17 g (0,0138 mole) de 2-chloropyrazine-5-carboxamide, 1,9 g (0,0138 mole) de carbonate de potassium et 100 ml d'acétonitrile, et on chauffe le mélange au reflux pendant 18h, on le laisse refroidir, on évapore le solvant sous pression réduite et on purifie le résidu par chromatographie sur colonne de gel de silice en éluant avec un mélange dichlorométhane/méthanol de 100/0 à 90/10. On recristallise le solide obtenu dans l'acétate d'éthyle, et on obtient 2,37 g (0,006 mole) de base.
On prépare le fumarate à partir de 2,37 g de base en solution dans 50 ml de méthanol et de 0,7 g (0,006 mole) d'acide fumarique en solution dans 50 ml de méthanol. On concentre le mélange sous pression réduite et le produit cristallise. On obtient 1,7 g de solide blanc.
Point de fusion : 184-186°C.

### Exemple 5 (Composé n°10).

(*E*)but-2-ènedioate de 2-[[3-[4-(5-chloro-2-méthoxyphényl)pipérazin-1-yl]propyl]amino]pyrazine-5-carboxamide (1:1).

### 5.1. 2-[3-[4-(5-Chloro-2-méthoxyphényl)pipérazin-1-yl]propyl)-1H-isoindole-1,3(2H)-dione.

Dans un ballon de 500 ml, on introduit 17,16 g (0,05236 mole) de (*E*) -but-2-ènedioate de 1-(5-chloro-2-méthoxyphényl)pipérazine (1:1), 14,04 g (0,05236 mole) de 2-(3-bromopropyl)-1*H*-isoindole-1,3(2*H*)-dione, 7,24 g (0,05236 mmole) de carbonate de potassium en suspension dans 150 ml de diméthylformamide, et on chauffe le mélange pendant 4h à 90°C.
On verse le mélange réactionnel sur 300 ml d'eau et on extrait à l'acétate d'éthyle (2 × 150 ml). On lave la phase organique à l'eau (3 × 150 ml) puis on la sèche sur sulfate de sodium, on filtre et on évapore les solvants sous pression réduite. On recristallise le résidu brut dans l'éther diéthylique et on obtient 14,7 g de solide.
Point de fusion : 130-131°C.

### 5.2. 3-[4-(5-Chloro-2-méthoxyphényl)pipérazin-1-yl]propanamine.

Dans un ballon de 1 l, on place 19,7 g (0,05105 mole) de 2-[3-[4-(5-chloro-2-méthoxyphényl)pipérazin-1-yl]propyl)-1*H*-isoindole-1,3(2*H*)-dione en solution dans 300 ml d'éthanol puis on additionne 5,11 g (0,1021 mole) d'hydrate d'hydrazine et on chauffe à la température de reflux pendant 4h.
On évapore le solvant sous pression réduite puis on ajoute au résidu brut 100 ml d'eau et 17 ml d'acide chlorhydrique concentré et on chauffe à nouveau à la température de reflux du solvant pendant 3h.
On sépare l'insoluble par filtration, on basifie le filtrat avec de la soude à 30%, puis on extrait au dichlorométhane. On lave la phase organique à l'eau, on la sèche sur sulfate de sodium, on la filtre puis on évapore les solvants sous pression réduite pour obtenir 13,76 g d'huile qu'on utilise telle quelle dans l'étape suivante.

### 5.3. (E)-but-2-ènedioate de 2-[[3-[4-(5-chloro-2-méthoxyphényl)pipérazin-1-yl]propyl]amino]pyrazine-5-carboxamide (1:1).

Dans un ballon de 500 ml, on introduit 13,67 g (0,04817 mole) de 3-[4-(5-chloro-2-méthoxyphényl)pipérazin-1-yl]propanamine, 8,65 g (0,062 mole) de carbonate de potassium, et 7,59 g (0,04817 mmole) de 2-chloropyrazine-5-carboxamide en suspension dans 200 ml de diméthylformamide, et on agite le mélange à température ambiante pendant 48h.
On évapore le solvant sous pression réduite, on purifie le résidu par recristallisation dans l'acétate d'éthyle et on obtient 12,6 g de base.
On prépare le fumarate à partir de 1,58 g (0,0039 mole) de base dans 50 ml d'éthanol et de 0,47 g (0,0039 mole) d'acide fumarique dans 50 ml d'éthanol. On concentre le mélange et on recristallise le produit dans un mélange méthanol/éthanol. On obtient finalement 1,08 g (0,00207 mole) de solide blanc.
Point de fusion : 219-223°C (décomposition).

### Exemple 6 (Composé n°13)

### [2-[[[2-[[3-[4-(5-Chloro-2-méthoxyphényl)pipérazin-1-yl]propyl]amino]pyrazin-5-yl]carbonyl]amino]éthyl]carbamate de 1,1-diméthyléthyle.

### 6.1 2-[[3-[4-(5-Chloro-2-méthoxyphényl)pipérazin-1-yl]propyl]amino]pyrazine-5-carboxylate de méthyle.

Dans un ballon de 1 l, on introduit 9,7 g (0,024 mole) de 2-[[3-[4-(5-chloro-2-méthoxyphényl)pipérazin-1-yl]propyl]amino]pyrazine-5-carboxamide dans 400 ml de méthanol, puis on fait passer un courant d'acide chlorhydrique gazeux pendant quelques minutes et on chauffe, à la température du reflux du méthanol pendant 5h.
On évapore le solvant sous pression réduite, on ajoute au résidu 200 ml de dichlorométhane et on refroidit à 0°C. On alcalinise le mélange avec une solution aqueuse saturée d'hydrogénocarbonate de sodium, on décante et sèche la phase organique sur sulfate de sodium, on filtre puis évapore le solvant sous pression réduite.
Après chromatographie sur colonne de silice (éluant : mélange dichlorométhane/méthanol de 100/0 à 90/0) puis recristallisation dans du cyclohexane, on isole 8,47 g (0,020 mole) de composé.
Point de fusion : 120-122°C.

### 6.2 [2-[[[2-[[3-[4-(5-Chloro-2-méthoxyphényl)pipérazin-1-yl]propyl]amino]pyrazin-5-yl]carbonyl]amino]éthyl]carbamate de 1,1-diméthyléthyle

Dans un ballon de 0,5 l, on introduit 4 g (0,0095 mole) de 2-[[3-[4-(5-chloro-2-méthoxyphényl)pipérazin-1-yl]propyl]amino]pyrazine-5-carboxylate de méthyle et 3,05 g (0,02 mole) de (2-aminoéthyl)carbamate de 1,1-diméthyléthyle dans 10 ml de propan-2-ol, et on chauffe le mélange au reflux pendant 2 jours.
On évapore le solvant sous pression réduite et on purifie par chromatographie sur colonne de gel de silice (éluant dichlorométhane/méthanol de 100/0 à 90/10) pour obtenir une huile jaune qui cristallise par trituration dans l'éther diéthylique. On isole finalement 1,5 g (0,00274 mole) de composé.
Point de fusion : 159-161°C.

### Exemple7 (Composé n°14).

### N-(2-Aminoéthyl)-2-[[3-[4-(5-chloro-2-méthoxyphényl)pipérazin-1-yl]propyl]amine]pyrazine-5-carboxamide.

Dans un ballon de 0,25 l, on introduit 2 g (0,00365 mole) de [2-[[[2-[[3-[4-(5-chloro-2-méthoxyphényl)pipérazin-1-yl]propyl]amino]pyrazin-5-yl]carbonyl]amino]éthyl]carbamate de 1,1-diméthyléthyle dans 10 ml d'eau puis, goutte à goutte 10 ml d'acide chlorhydrique concentré. On refroidit le mélange à 0°C avec un mélange glace/sel/eau, on ajoute par portions de la soude à 30% jusqu'à pH basique. On extrait au dichlorométhane, on sèche la phase organique sur sulfate de sodium, on filtre et évapore les solvants sous pression réduite, et on obtient 1,32 g (0,00295 mole) de solide amorphe.
Point de fusion : 45-55°C.

### Exemple 8 (Composé n°15).

### N-[2-[[4-Aminocarbonyl)pyrimidin-2-yl]amino]éthyl]-2-[[3-[4-(5-chloro-2-méthoxyphényl)pipérazin-1-yl]propyl]amino]pyrazine-5-carboxamide

Dans un ballon de 0,25 l, on introduit 1,32 g (0,00295 mole) de *N*-(2-Aminoéthyl)-2-[[3-[4-(5-chloro-2-méthoxyphényl)pipérazin-1-yl]propyl]amino]pyrazine-5-carboxamide, 0,5 g (0,00317 mole) de 2-chloropyrimidine-4-carboxamide, 0,6 g (0,00434 mole) de carbonate de potassium dans 50 ml de diméthylformamide et on chauffe à 40°C pendant 40h.
On évapore le solvant sous pression réduite et on purifie le résidu brut par chromatographie sur colonne de gel de silice en éluant avec un mélange dichlorométhane/méthanol de 98/2 à 80/20. Après recristallisation dans l'acétonitrile on obtient finalement 0,99 g (0,00174 mole) de composé.
Point de fusion : 197-199°C.

Le tableau qui suit illustre les structures chimiques et les propriétés physiques de quelques composés selon l'invention.

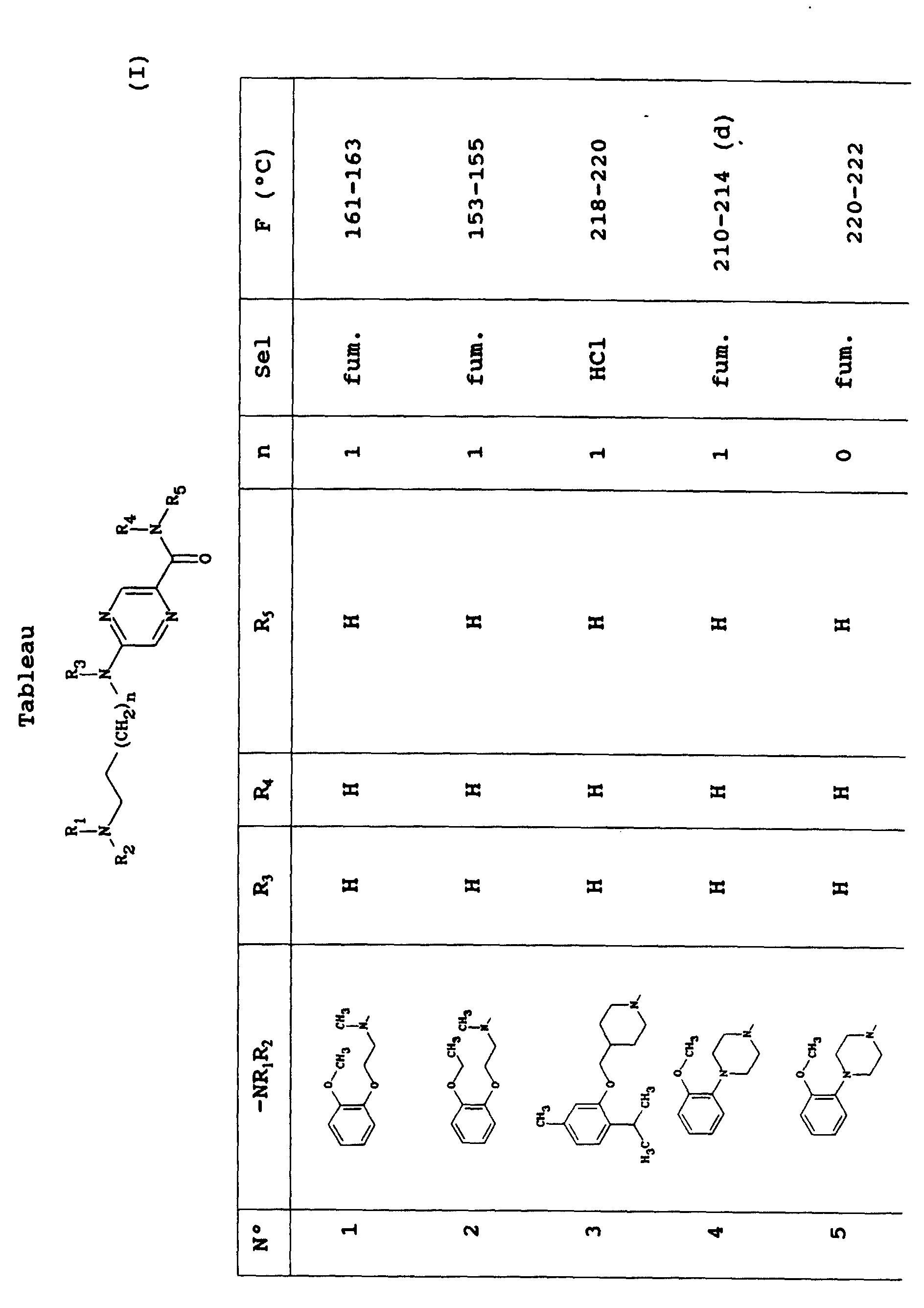

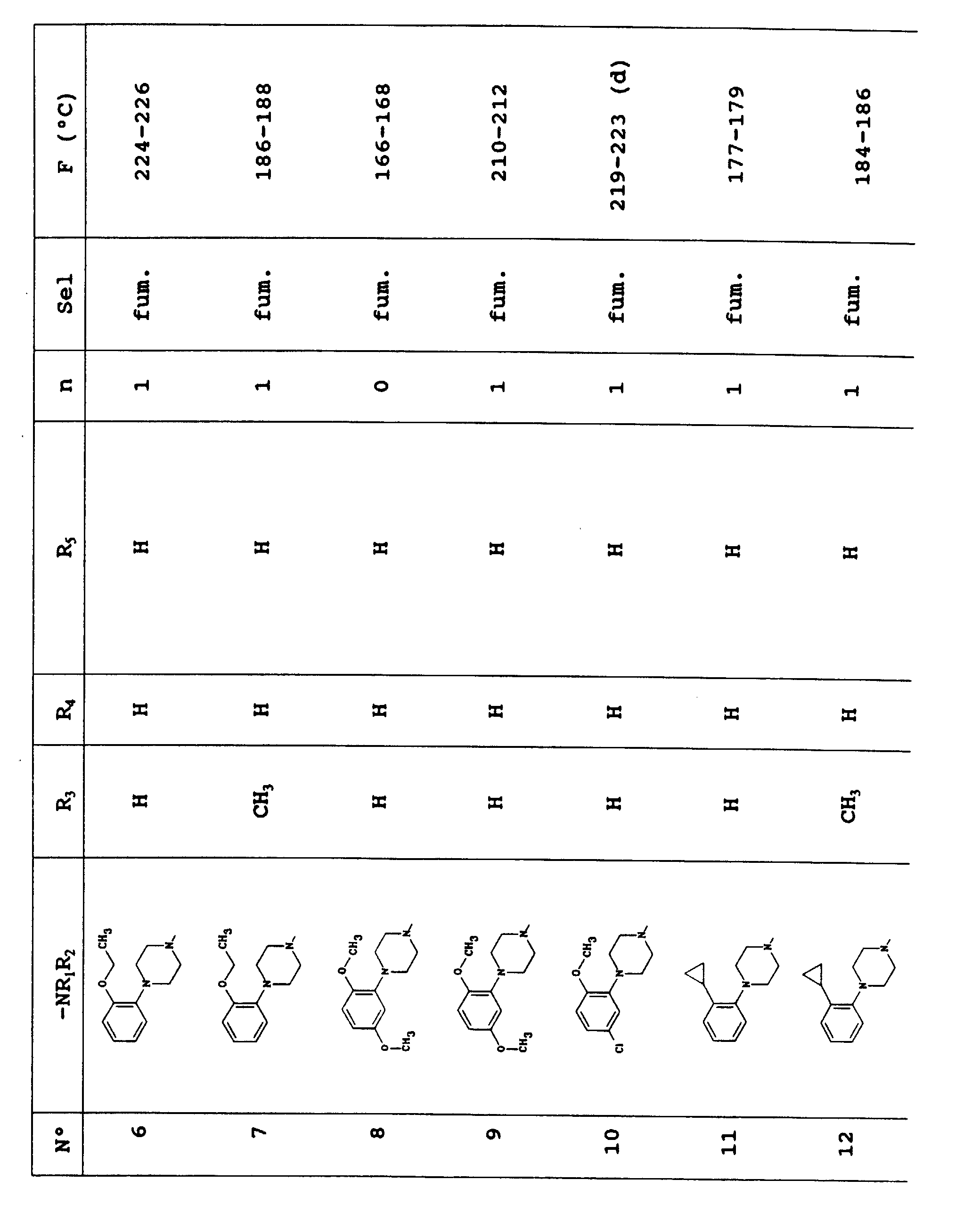

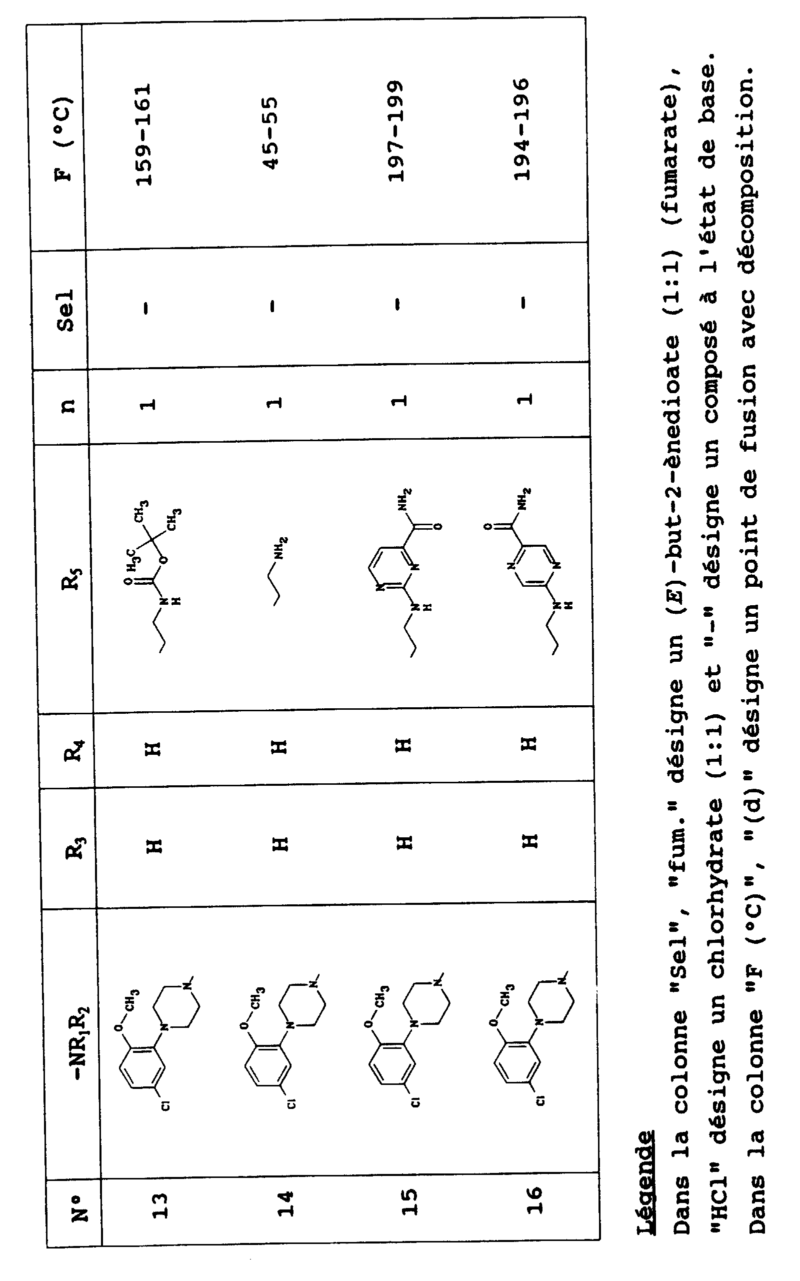

Les composés de l'invention ont fait l'objet d'études quant à leur activité antagoniste des récepteurs α1-adrénergiques au niveau du bas appareil urinaire.

Leur activité *in vitro* a été étudiée sur l'urètre isolé de lapin.
On prépare des anneaux d'urètre de lapin mâle adulte selon la méthode de Ueda et al., *Eur. J. Pharmacol.*, (1984), **103**, 249-254, puis, après sensibilisation à la noradrénaline, on détermine la courbe concentration-réponse à la phényléphrine, en absence et en présence de composé à étudier.
On évalue la puissance de l'antagonisme α₁-adrénergique de chaque composé par calcul du pA₂, antilogarithme de la concentration molaire d'antagoniste en présence de laquelle la concentration d'agoniste doit être doublée pour engendrer le même effet qu'en son absence.
Les pA₂ des composés sont compris entre 7 et 10.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.*, (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme α₁-adrénergique de chaque composé par calcul de la DI₅₀, dose qui inhibe de 50% l'hypertonie urétrale.
Les DI₅₀ des composés de l'invention sont comprises entre 0,001 et 1 mg/kg.

Les résultats des essais montrent que les composés de l'invention montrent, *in vitro*, une activité antagoniste des récepteurs α₁-adrénergiques des muscles lisses du bas appareil urinaire (urètre) stimulés par un agoniste α₁-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système α-adrénergique au niveau du bas appareil urinaire, et notamment pour le traitement des troubles mictionnels de l'hypertrophie bénigne de la prostate, tels que la dysurie et la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,1 à 500 mg de substance active.

## Revendications

1. Composé répondant à la formule générale (I) 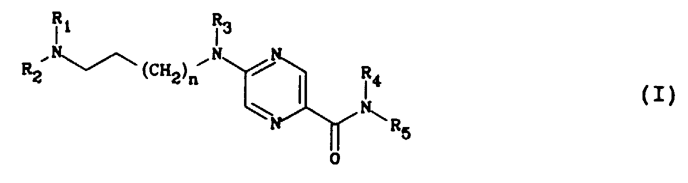 dans laquelle
n représente le nombre 0 ou 1,
R₁ représente un groupe méthyle, auquel cas
R₂ représente un groupe phénoxy(C₁-C₄)alkyle (dont le groupe phénoxy porte éventuellement 1 ou 2 substituants choisis parmi les atomes d'halogène et les groupes méthoxy et éthoxy), ou bien R₁ et R₂ forment ensemble, et avec l'atome d'azote qui les porte, un groupe 4-(phénoxyméthyl)pipéridin-1-yle (dont le groupe phénoxy porte éventuellement 1 ou 2 groupes alkyles en C₁-C₄), ou un groupe 4-phénylpipérazin-1-yle (dont le groupe phényle porte éventuellement 1 ou 2 substituants choisis parmi les atomes d'halogènes et les groupes méthoxy et éthoxy et les groupes alkyles en C₁-C₄),
R₃ représente un atome d'hydrogène ou un groupe méthyle,
R₄ représente un atome d'hydrogène,
R₅ représente un atome d'hydrogène ou un groupe de formule générale 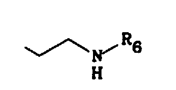
R₆ étant un atome d'hydrogène, un groupe tertiobutyloxycarbonyle, un groupe 4-carbamoylpyrimidin-2-yle ou un groupe 5-carbamoylpyrazin-2-yle,
à l'état de base ou de sel d'addition à un acide.

2. Procédé de préparation de composés selon la revendication 1, caractérisé en ce qu'on fait réagir une amine de formule générale (II) 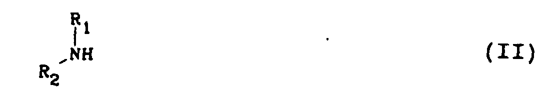 (dans laquelle R₁ et R₂ sont tels que définis ci-dessus) éventuellement sous forme de sel, avec un réactif halogéné de formule générale (III) 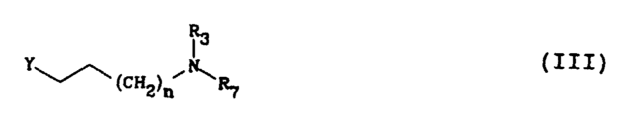 (dans laquelle Y représente un atome d'halogène, n est tel que défini dans la revendication 1, et soit R₃ est tel que défini dans la revendication 1 et R₇ représente un groupe triphénylméthyle, soit R₃ et R₇ forment ensemble, et avec l'atome d'azote qui les porte, un groupe phtalimido) dans un solvant aprotique, en présence d'une base minérale, à une température de 40 à 80°C,
obtenant ainsi une diamine de formule générale (IV) 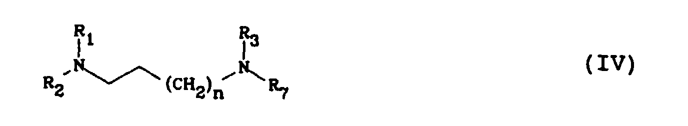 dont on déprotège l'alkylamine terminale : dans le cas où R₇ est un groupe triphénylméthyle, on effectue un traitement à l'acide chlorydrique gazeux dans un alcool aliphatique, à une température de 0 à 60°C ; dans le cas où R₃ et R₇ forment ensemble un groupe phtalimido, on effectue un traitement avec l'hydrazine,
obtenant ainsi une amine de formule générale (V) 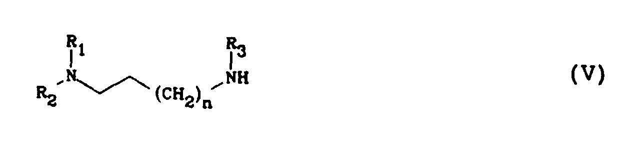 que l'on fait réagir avec le 2-chloropyrazine-5-carboxamide dans un solvant aprotique, en présence d'une base, à une température de 20 à 40°C,
obtenant ainsi un dérivé de 2-aminopyrazine-5-carboxamide de formule générale (Ia) 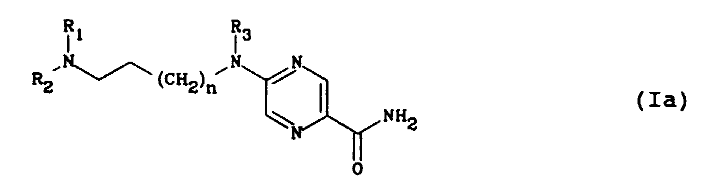 qui correspond à la formule générale (I) lorsque R₄ et R₅ représentent chacun un atome d'hydrogène,
puis, pour préparer les composés de formule générale (I) dans laquelle R₅ représente un groupe de formule générale 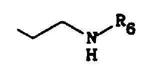 on transforme l'amide de formule générale (Ia) en ester de formule générale (VII) 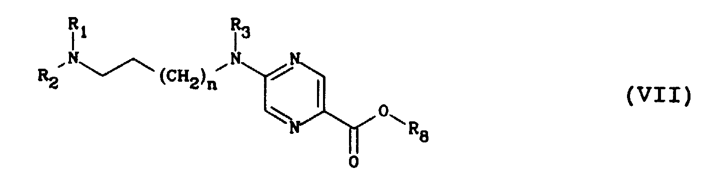 (dans laquelle R₈ représente un groupe alkyle en C₁-C₄) par réaction avec un alcool aliphatique en C₁-C₄, en présence d'un acide, à une température de 0 à 60°C, puis on fait réagir l'ester ainsi obtenu avec une diamine de formule générale (VIII) 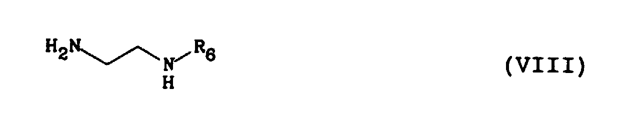 (dans laquelle R₆ représente un groupe tertiobutyloxycarbonyle) dans un alcool aliphatique, à une température de 0 à 100°C,
obtenant ainsi un composé de formule générale (Ib) 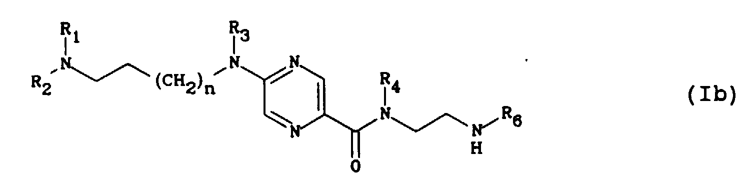 dans laquelle R₆ représente un groupe tertiobutyloxycarbonyle, puis, pour préparer les composés de formule générale (Ib) dans laquelle R₆ représente un groupe 4-carbamoylpyrimidin-2-yle- ou 5-carbamoylpyrazin-2-yle, on déprotège le composé précédemment obtenu par l'acide trifluoroacétique dans le dichlorométhane, pour obtenir un composé de formule générale (Ib) où R₆ représente l'hydrogène, et on fait réagir ce dernier avec le 2-chloropyrimidine-4-carboxamide où le 2-chloropyrazine-5-carboxamide, dans un solvant aprotique, en présence d'une base, à une température de 20 à 40°C.

3. Médicament caractérisé en ce qu'il consiste en un composé selon la revendication 1.

4. Composition pharmaceutique, caractérisée en ce qu'elle contient un composé selon la revendication 1, associé à un excipient.

5. Composé répondant à la formule générale (VII) 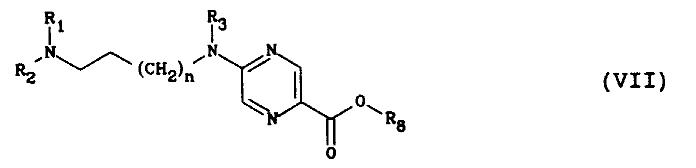 dans laquelle n, R₁, R₂ et R₃ sont tels que définis dans la revendication 1 et R₈ représente un groupe alkyle en C₁-C₄, à titre d'intermédiaire nécessaire dans le procédé selon la revendication 2.

## Patentansprüche

1. Verbindung der allgemeinen Formel (I) 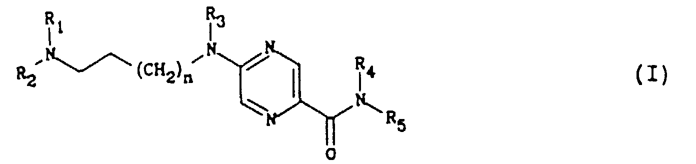 in der
n die Zahl 0 oder 1,
R₁ eine Methylgruppe, in welchem Fall
R₂ eine Phenoxy-(C₁-C₄)-alkylgruppe (deren Phenoxygruppe gegebenenfalls 1 oder 2 Substituenten ausgewählt aus Halogenatomen, Methoxygruppen und Ethoxygrupen trägt) oder R₁ und R₂ gemeinsam mit dem sie tragenden Stickstoffatom eine 4-(Phenoxymethyl)-piperidin-1-yl-gruppe (deren Phenoxygruppe gegebenenfalls 1 oder 2 C₁-C₄-Alkylgruppen trägt) oder eine 4-Phenylpiperazin-1-yl-gruppe (deren Phenlygruppe gegebenenfalls 1 oder 2 Substituenten ausgewählt aus Halogenatomen, Methoxygruppen und Ethoxygruppen und C₁-C₄-Alkylgruppen trägt), bilden
R₃ ein Wasserstoffatom oder eine Methylgruppe,
R₄ ein Wasserstoffatom,
R₅ ein Wasserstoffatom oder eine Gruppe der allgemeinen Formel 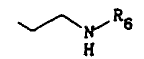 in der R₆ ein Wasserstoffatom, eine tert.-Butyloxycarbonylgruppe, eine 4-Carbamoylpyrimidin-2-yl-gruppe oder eine 5-Carbamoylpyrazin-2-yl-gruppe darstellt, bedeuten,
in Form der Base oder des Additionssalzes mit einer Säure.

2. Verfahren zur Herstellung der Verbindungen nach Anspruch 1, dadurch gekennzeichnet, daß man ein Amin der allgemeinen Formel (II) 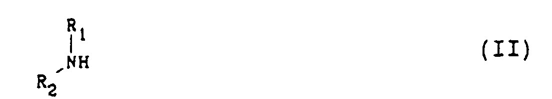 (in der R₁ und R₂ die oben angegebenen Bedeutungen besitzen) gegebenenfalls in Form des Salzes mit einem halogenierten Reaktionspartner der allgemeinen Formel (III) 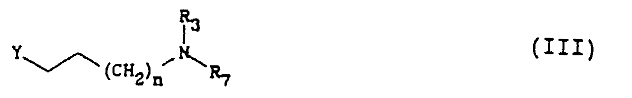 (in der Y ein Halogenatom darstellt und n die in Anspruch 1 angegebenen Bedeutungen besitzt und entweder R₃ die in Anspruch 1 angegebenen Bedeutungen besitzt und R₇ eine Triphenylmethylgruppe darstellt oder R₃ und R₇ gemeinsam mit dem sie tragenden Stickstoffatom eine Phthalimldogruppe bilden) in einem aprotischen Lösungsmittel in Gegenwart einer anorganischen Base bei einer Temperatur von 40 bis 80°C umsetzt,
so daß man ein Diamin der allgemeinen Formel (IV) erhält 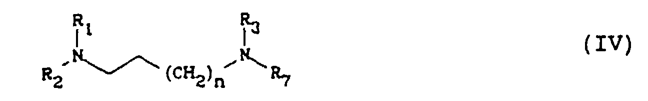 von dem man die Schutzgruppe der endständigen Alkylaminogruppe abspaltet: wobei in dem Fall, da R₇ eine Trlphenylmethylgruppe darstellt, man eine Behandlung mit gasförmiger Chlorwasserstoffsäure in einem aliphatischen Alkohol bei einer Temperatur von 0 bis 60°C durchführt; und in dem Fall, da R₃ und R₇ gemeinsam eine Phthalimidogruppe bilden, man eine Behandlung mit Hydrazin durchführt,
so daß man ein Amin der allgemeinen Formel (V) erhält 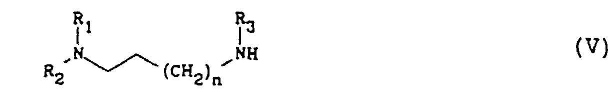 welches man mit 2-Chlorpyrazin-5-carboxamid in einem aprotischen Lösungsmittel und in Gegenwart einer Base bei einer Temperatur von 20 bis 40°C umsetzt, so daß man in dieser Weise ein 2-Aminopyrazin-5-carboxamid-Derivat der allgemeinen Formel (Ia) erhält 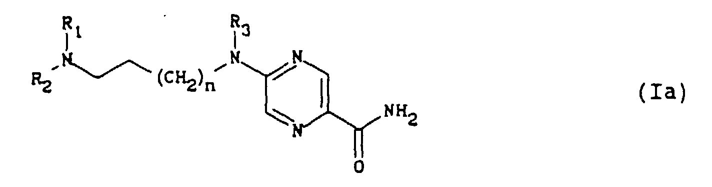 welche der allgemeinen Formel (I) entspricht, wenn R₄ und R₅ jeweils ein Wasserstoffatom bedeuten,
worauf man zur Herstellung der Verbindungen der allgemeinen Formel (I), in der R₅ eine Gruppe der allgemeinen Formel 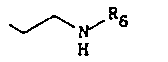 darstellt, man das Amid der allgemeinen Formel (Ia) durch Reaktion mit einem aliphatischen C₁-C₄-Alkohol in Gegenwart einer Säure bei einer Temperatur von 0 bis 60°C in den Ester der allgemeinen Formel (VII) umwandelt 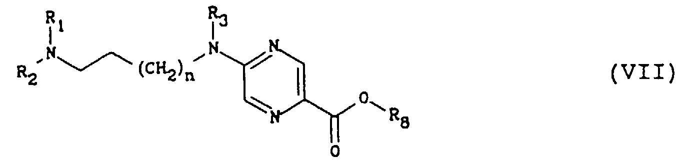 (in der R₈ eine C₁-C₄-Alkylgruppe darstellt), wonach man den in dieser Weise erhaltenen Ester mit einem Diamin der allgemeinen Formel (VIII) 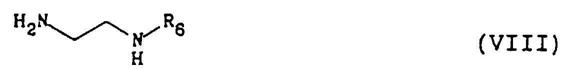 (in der R₆ eine tert. -Butyloxycarbonylgruppe darstellt) in einem aliphatischen Alkohol bei einer Temperatur von 0 bis 100°C umsetzt,
zur Bildung einer Verbindung der allgemeinen Formel (Ib) 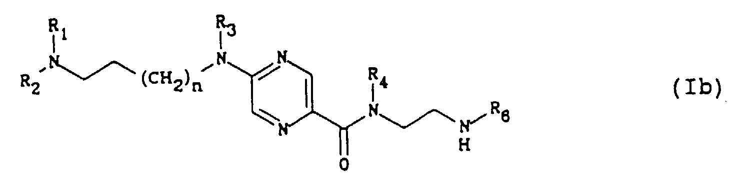 in der R₆ eine tert.-Butyloxycarbonylgruppe darstellt, wonach man zur Herstellung der Verbindungen der allgemeinen Formel (Ib), in der R₆ eine 4-Carbamoylpyrimidin-2-yl- oder 5-Carbamoylpyrazin-2-yl-gruppe bedeutet, man die Schutzgruppe der in der obigen Weise erhaltenen Verbindung mit Trifluoressigsäure in Dichlormethan abspaltet zur Bildung einer Verbindung der allgemeinen Formel (Ib), in der R₆ ein Wasserstoffatom darstellt, und man die letztere Verbindung mit 2-Chlorpyrimidin-4-carboxamid oder 2-Chlorpyrazin-5-carboxamid in einem aprotischen Lösungsmittel in Gegenwart einer Base bei einer Temperatur von 20 bis 40°C umsetzt.

3. Arzneimittel, dadurch gekennzeichnet, daß es aus einer Verbindung nach Anspruch 1 besteht.

4. Pharmazeutische Zubereitung, dadurch gekennzeichnet, daß sie eine Verbindung nach Anspruch 1 in Kombination mit einem Trägermaterial enthält.

5. Verbindung der allgemeinen Formel (VII) 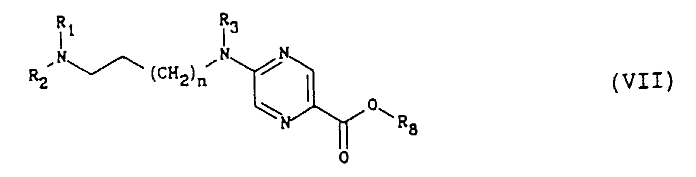 in der n, R₁, R₂ und R₃ die in Anspruch 1 angegebenen Bedeutungen besitzen und R₈ eine C₁-C₄-Alkylgruppe darstellt, als Zwischenprodukte für das Verfahren gemäß Anspruch 2.

## Claims

1. Compound corresponding to the general formula (I) 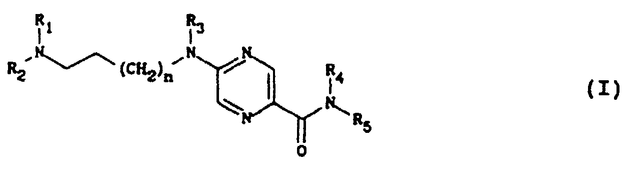 in which
n represents the number 0 or 1,
R₁ represents a methyl group, in which case
R₂ represents a phenoxy(C₁-C₄)alkyl group (in which the phenoxy group optionally carries 1 or 2 substituents chosen from halogen atoms and methoxy and ethoxy groups), or else R₁ and R₂ together form, and with the nitrogen atom which carries them, a 4-(phenoxymethyl)piperid-1-yl group (in which the phenoxy group optionally carries 1 or 2 C₁-C₄ alkyl groups) or a 4-phenylpiperazin-1-yl group (in which the phenyl group optionally carries 1 or 2 substituents chosen from halogen atoms and methoxy and ethoxy groups and C₁-C₄ alkyl groups),
R₃ represents a hydrogen atom or a methyl group,
R₄ represents a hydrogen atom,
R₅ represents a hydrogen atom or a group of general formula 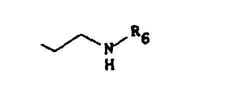
R₆ being a hydrogen atom, a tert-butyloxycarbonyl group, a 4-carbamoylpyrimidin-2-yl group or a 5-carbamoylpyrazin-2-yl group,
in the form of the base or of an addition salt with an acid.

2. Process for the preparation of compounds according to Claim 1, characterized in that an amine of general formula (II) 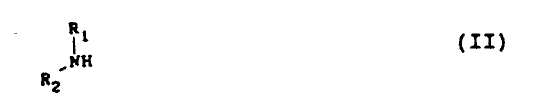 (in which R₁ and R₂ are as defined above), optionally in the salt form, is reacted with a halogenated reactant of general formula (III) 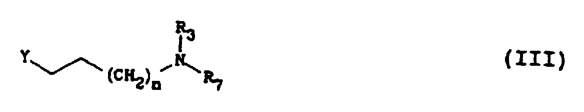 (in which Y represents a halogen atom, n is as defined in Claim 1 and either R₃ is as defined in Claim 1 and R₇ represents a triphenylmethyl group or R₃ and R₇ together form, and with the nitrogen atom which carries them, a phthalimido group) in an aprotic solvent, in the presence of an inorganic base, at a temperature of 40 to 80°C,
thus obtaining a diamine of general formula (IV) 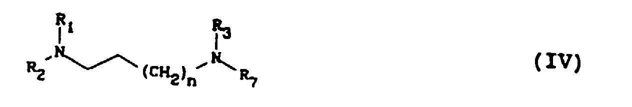 the end alkylamine of which is deprotected: in the case where R₇ is a triphenylmethyl group, a treatment with gaseous hydrochloric acid in an aliphatic alcohol at a temperature of 0 to 60°C is carried out; in the case where R₃ and R₇ together form a phthalimido group, a treatment with hydrazine is carried out,
thus obtaining an amine of general formula (V) 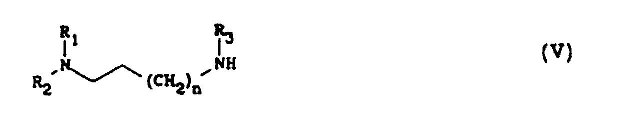 which is reacted with 2-chloropyrazine-5-carboxamide in an aprotic solvent, in the presence of a base, at a temperature of 20 to 40°C,
thus obtaining a 2-aminopyrazine-5-carboxamide derivative of general formula (Ia) 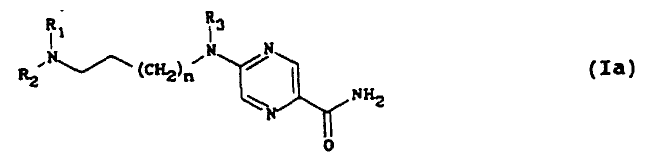 which corresponds to the general formula (I) when R₄ and R₅ each represent a hydrogen atom,
then, in order to prepare the compounds of general formula (I) in which R₅ represents a group of general formula 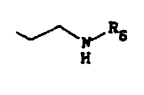 the amide of general formula (Ia) is converted to an ester of general formula (VII) 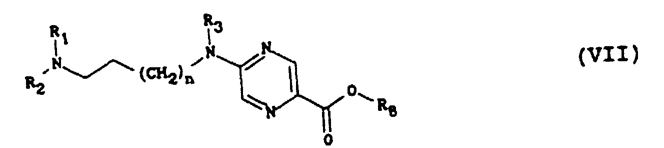 (in which R₈ represents a C₁-C₄ alkyl group) by reaction with a C₁-C₄ aliphatic alcohol, in the presence of an acid, at a temperature of 0 to 60°C, then the ester thus obtained is reacted with a diamine of general formula (VIII) 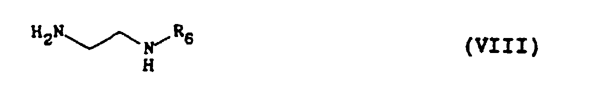 (in which R₆ represents a tert-butyloxycarbonyl group) in an aliphatic alcohol, at a temperature of 0 to 100°C, thus obtaining a compound of general formula (Ib) 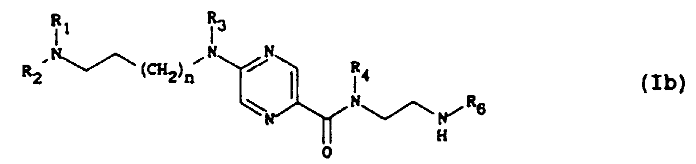 in which R₆ represents a tert-butyloxycarbonyl group, then, in order to prepare the compounds of general formula (Ib) in which R₆ represents a 4-carbamoylpyrimidin-2-yl or 5-carbamoylpyrazin-2-yl group, the compound obtained above is deprotected with trifluoroacetic acid in dichloromethane in order to obtain a compound of general formula (Ib) where R₆ represents hydrogen, and the latter is reacted with 2-chloropyrimidine-4-carboxamide or 2-chloropyrazine-5-carboxamide, in an aprotic solvent, in the presence of a base, at a temperature of 20 to 40°C.

3. Medicament, characterized in that it consists of a compound according to Claim 1.

4. Pharmaceutical composition, characterized in that it contains a compound according to Claim 1, in combination with an excipient.

5. Compound corresponding to the general formula (VII) 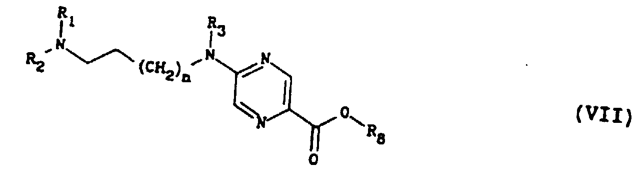 in which n, R₁, R₂ and R₃ are as defined in Claim 1 and R₈ represents a C₁-C₄ alkyl group, as an essential intermediate in the process according to Claim 2.
